# EUROPÄISCHE PATENTSCHRIFT

(11) **EP 1 285 910 B1**
(45) Veröffentlichungstag und Bekanntmachung des Hinweises auf die Patenterteilung: **07.06.2006**
(21) Anmeldenummer: 02017347.2
(22) Anmeldetag: 02.08.2002
(51) Int. Cl.: C07C 303/22, C07C 303/32, C07C 309/42

(54) **Verfahren zur Herstellung von Acyloxybenzolsulfonaten**
Process for preparing acyloxybenzenesulfonates
Procédé de préparation d'acyloxybenzènesulfonates

(30) Priorität: 11.08.2001 DE 10139663
(43) Veröffentlichungstag der Anmeldung: 26.02.2003
(73) Patentinhaber: Clariant Produkte (Deutschland) GmbH, 65929 Frankfurt am Main (DE)
(72) Erfinder: Reinhardt, Gerd, Dr., 65779 Kelkheim (DE); Naumann, Peter, Dl., 65232 Taunusstein (DE); Ladwig, Miriam, 63128 Dietzenbach (DE); Golla, Ina, 65428 Rüsselsheim (DE); Pilz, Torsten, Dr., 61389 Oberreifenberg (DE); Wieduwilt, Retro, Dipl.-Chem., 4303 Kaiseraugst (CH); Jourdan, Henri, Dipl.-Chem., 4052 Basel (CH)

(56) Entgegenhaltungen:
- EP-A- 0 164 786
- US-A- 4 883 612

## Beschreibung

Die Erfindung betrifft ein Verfahren zur Herstellung von Acyloxybenzolsulfonaten, ausgehend von Carbonsäurehalogeniden und wasserarmen Salzen der Phenolsulfonsäure.

Acyloxybenzolsulfonsäuren und ihre Salze sind seit langem bekannte Verbindungen. In Abhängigkeit von der Kettenlänge der Acylgruppe können sie als Tenside, Bleichaktivatoren oder in anderen Anwendungen Verwendung finden. Diese Verbindungen können erhalten werden durch Reaktion von Natriumphenolsulfonat (SPS) mit dem Chlorid einer organischen Carbonsäure. Als Reaktionsmedium dienen organische Lösungsmittel wie Methylenchlorid (US 35 03 888), hochsiedende Kohlenwasserstoffe (EP 220 826), Xylol oder Toluol (EP 164 786). Gemäß US 5 069 828 wird diese Reaktion in einem aprotischen organischen Lösungsmittel in Anwesenheit eines Phasentransferkatalysators durchgeführt.

WO 01/19 771 beschreibt die Umsetzung von Acylchloriden mit SPS in Trifluoressigsäure (TFA) als Lösemittel. In den Beispielen wird mit einem Verhältnis TFA : SPS von 0,5 : 1 bis 3 : 1 gearbeitet. Der hohe Preis der TFA, ihre vollständige Abtrennung vom Reaktionsgemisch, sowie mögliche Umesterungsreaktionen bzw. Nebenproduktbildung stehen einer großtechnischen Nutzung des Verfahrens allerdings entgegen.

Bei allen Verfahren ergibt sich das Problem, dass für die Reaktion nahezu wasserfreies SPS benutzt werden muss, da andernfalls Acylhalogenid bzw. der fertige Ester in Gegenwart von Wasser hydrolysiert werden, was zu erheblichen Ausbeuteverlusten führt. SPS ist kommerziell als Dihydrat mit einem Wasseranteil von ca. 15 % verfügbar. Durch konventionelle Trocknung kann der Wassergehalt auf ca. 2 % gesenkt werden. Nach US 5,069,828 ist es möglich, die Restwassermenge durch azeotrope Destillation in Gegenwart eines Schleppmittels wie Xylol zu entfernen. Wegen des hohen Zeitbedarfs ist dies aber in kontinuierlich arbeitenden Anlagen wenig sinnvoll. Alternativ kann der Wassergehalt durch spezielle Trocknung in entsprechenden Apparaturen auf unter 1 % gesenkt werden. Wie jedoch aus US 4,666,636 bekannt ist, müssen bestimmte Trocknungsbedingungen exakt eingehalten werden, da SPS sonst eine Reihe von Nebenreaktionen eingehen kann, wodurch sowohl der Umsetzungsgrad der folgenden Acylierung, wie auch die Farbe des Endprodukte signifikant negativ beeinflusst wird. Übertrocknung des SPS führt in der folgenden Acylierungsreaktion zu Umsetzungsgraden kleiner 50 %.

Aufgabe der vorliegenden Erfindung ist es deshalb, ein sowohl großtechnisch als auch kontinuierlich durchführbares Verfahren zu entwickeln, das in sehr guten Ausbeuten zu möglichst einheitlichen Produkten führt, die hinsichtlich Zusammensetzung, Qualität und Farbe für den Einsatz in Wasch- und Reinigungsmitteln geeignet sind. Dabei soll das Verfahren unabhängig von der Qualität des eingesetzten Natrium-Phenolsulfonats und dessen Vorbehandlung sein. Überraschenderweise wurde nun gefunden, dass Acyloxybenzolsulfonate in hohen Ausbeuten und guten Qualitäten, unabhängig von der Qualität des eingesetzten SPS, hergestellt werden können, wenn die Umsetzung des SPS mit einem Alkancarbonsäure halogenid in einem aliphatischen oder aromatischen Lösemittel in Gegenwart katalytischer Mengen eines offenkettigen oder cyclischen tertiären Amins oder Carbonsäureamids durchgeführt wird.

Gegenstand der Erfindung ist ein Verfahren zur Herstellung von Acyloxybenzolsulfonaten durch Umsetzung von Phenolsulfonaten, die einen Wassergehalt von weniger als 0,5, vorzugsweise weniger als 0,2 Gew.-% Wasser aufweisen, mit Alkancarbonsäure halogeniden, in einem aliphatischen oder aromatischen Kohlenwasserstoff, dadurch gekennzeichnet, dass man die Reaktion in Gegenwart von 0,01 bis 10 Gew.-% eines Katalysators in Form eines offenkettigen oder cyclischen tertiären Amins oder Carbonsaureamids durchführt.

Bei den als Ausgangsverbindungen dienenden Phenolsulfonaten handelt es sich vorzugsweise um Verbindungen der Formel 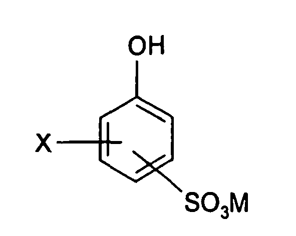 wobei X Wasserstoff, Halogen oder C₁-C₄-Alkyl und M ein Alkali- oder Erdalkalimetall-ion bedeutet.
Bevorzugt sind Natrium-ortho- oder para-Phenolsulfonate, insbesondere Natriumpara-phenolsulfonat (SPS), das herstellbedingt 0 bis 20 % des entsprechenden ortho-Isomeren enthalten kann.

SPS ist kommerziell als Dihydrat, d.h. mit einem Wassergehalt von 15 % verfügbar. Für die erfindungsgemäße Reaktion mit einem Alkancarbonsäurederivat muss das Phenolsulfonat zunächst auf eine Restfeuchte von höchstens 0,5, vorzugsweise höchstens 0,2 Gew.-% Wasser getrocknet werden. Dies kann nach üblichen und an sich bekannten Verfahren geschehen, beispielsweise in einem Scheibentrockner, der eine Trocknung auf eine Restfeuchte von weniger als 0,1 Gew.-% erlaubt.

Die Trockenzeiten können in Abhängigkeit von der verwendeten Ausrüstung zwischen 1 min und 18 h liegen, die Temperaturen zwischen 80 und 250°C. Bei dem erfindungsgemäßen Verfahren hat die Qualität des getrockneten SPS keinen Einfluss auf die Ausbeute der Acylierungsreaktion und es können im Schnitt Umsätze größer 95 % erzielt werden. Insbesondere können auch Trockenbedingungen angewandt werden, die außerhalb der in US 4,666,636 genannten optimalen Trockenbedingungen liegen, d.h. solche, die zu "übertrocknetem" Produkt führen. Ein solches Produkt kann gemäß dem Stand der Technik, nicht für Acylierungsreaktionen verwendet werden, da es nicht reaktiv genug ist.

Als Alkancarbonsäure halogenide kommen insbesondere die Carbonsäurechloride oder -bromide in Frage, wobei die Chloride bevorzugt sind. Diese können aus den entsprechenden Carbonsäuren z.B. durch Umsetzung mit Phosgen, Thionylchlorid, Phosphortrichlorid, Phosphoroxychlorid, Phosphorpentachlorid oder Phosphortribromid hergestellt werden.

Carbonsäure halogenid und Phenolsulfonat gemäß der Erfindung können vorzugsweise im Molverhältnis von 0,9 : 1 bis 2 : 1, vorzugsweise 1 : 1 bis 1,5 : 1 miteinander umgesetzt werden.

Als Reaktionsmedium dienen aliphatische oder aromatische Kohlenwasserstoffe mit Siedepunkten zwischen 80 und 220°C, insbesondere 100 bis 180°C, z.B. Toluol, Xylol, Paraffine mit 8 bis 22 Kohlenstoffatomen, wie Decan, Undecan, Dodecan, Hexadecan oder Octadecan
oder deren Mischungen. Besonders geeignet sind aliphatische Kohlenwasserstoffgemische wie sie als Shellsole (Shell), ISOPAR G und ISOPAR 4 (ESSO) kommerziell verfügbar sind. Die Löslichkeit des SPS in diesem Reaktionsmedium liegt häufig unter 1 %.

Der Katalysator werden offenkettige oder cyclische tertiäre Amine oder Carbonsäureamid verwendet . Verwendung finden können Triethylamin, Tripropylamin, Dimethylformamid, Diethylformamid, Dimethylacetamid, Tetramethylharnstoff, subsituierte Pyrrolidone wie N-Methylpyrrolidon, N-Ethylpyrrolidon, N-Butylpyrrolidon, N-Octylpyrrolidon, n-Methylcaprolactam, N-Octylcaprolactam. Besonders bevorzugt sind Triethylamin, Dimethylformamid, N-Methylpyrrolidon und N-Octylpyrrolidon. Das Molverhältnis des eingesetzten Katalysators zum Phenolsulfonat beträgt 0,0001 : 1 bis 0,02 : 1, vorzugsweise 0,005 : 1 bis 0,012 : 1.

Die Acylierungsreaktion wird bei Temperaturen zwischen 60 und 200°C, insbesondere zwischen 100 und 150°C durchgeführt. Das während der Reaktion entstehende Gas wird abgeleitet, ggf. wird die Reaktion mit einem inerten Gasstrom aus Stickstoff oder Argon überlagert. Die Reaktion wird im Sinne einer heterogenen Reaktion (Slurry) durchgeführt, da weder das Phenolsulfonat noch das entstehende Acyloxybenzolsulfonat eine nennenswerte Löslichkeit im Reaktionsmedium aufweisen. Die Reaktionszeit richtet sich nach den Reaktionsbedingungen und kann zwischen 10 min und 5 h, vorzugsweise 30 bis 120 min betragen.

In einer besonderen Ausführungsform kann die erfindungsgemäße Reaktion kontinuierlich durchgeführt werden. Hierzu besonders geeignet sind Kesselkaskaden bzw. Rohrreaktoren, wie sie dem Fachmann bekannt sind.

Nach beendeter Reaktion wird das Reaktionsprodukt mittels konventioneller Trennmethoden isoliert. Hierzu eignen sich Zentrifugen, Filterapparate. Zur völligen Abtrennung des Katalysators empfiehlt es sich, das feste Reaktionsprodukt ein oder mehrmals mit dem Reaktionsmedium auszuwaschen. Die Mutterlauge kann ohne weitere Reinigung für die nächsten Reaktionen verwendet bzw. cycliert werden. Das gebildete Acyloxybenzolsulfonat fällt in hohen Ausbeuten in Form eines weißen Pulvers an, das durch konventionelle Trocknung isoliert werden kann.

Das auf diese Weise gewonnene Acyloxybenzolsulfonat kann als Tensid oder Persalzaktivator in Wasch- und Reinigungsmittel wie pulverförmigen Vollwaschmitteln, Fleckensalzen oder pulverförmigen Maschinengeschirrspülmitteln eingesetzt werden. Zur Erhöhung der Lagerstabilität in diesen Formulierungen kann es, wie dem Fachmann bekannt, in eine granulare Form überführt werden.

### Beispiele

### Beispiel 1 a: Herstellung von wasserfreiem 4-Phenolsulfonat-Natrium (gute Qualität).

1 kg 4-Phenolsulfonat-Natrium mit einem Wassergehalt von 2,6 % wurden analog Beispiel 1 aus US 4,666,636 getrocknet. Man erhielt 975 g 4-Phenolsulfonat-Natrium mit einem Wassergehalt von< 0,1 %.

### Beispiel 1b: Herstellung von wasserfreiem 4-Phenolsulfonat-Natrium (schlechte Qualität)

1 kg 4-Phenolsulfonat-Natrium mit einem Wassergehalt von 2,6 % wurden 12 h bei 180°C getrocknet. Man erhielt 975 g 4-Phenolsulfonat-Natrium mit einem Wassergehalt von < 0,1 %.

### Vergleichsbeispiel A: Verwendung von SPS, getrocknet gemäß Beispiel 1a

98,1 g (0,5 mol) getrocknetes Phenolsulfonat-Natrium, hergestellt nach Beispiel 1 wurden in 150 g ISOPAR G vorgelegt und auf 120°C erwärmt. Anschließend wurden 114,8 g (0,65 mol) Nonansäurechlorid innerhalb von 30 min zugetropft und bei 130°C nachgerührt. Das dabei entstehende HCI-Gas wurde abgeleitet. Das Reaktionsgemisch wurde nach 2 h auf 80°C abgekühlt und über einen Filter abfiltriert. Das weiße Reaktionsprodukt wurde 2 mal mit wenig ISOPAR G nachgewaschen und anschließend im Trockenschrank bei 110 - 130°C über Nacht getrocknet.
Telquel-Ausbeute: 164,8 g (Ausbeute 98 %) weißen Pulvers mit einem Nonanoyloxybenzolsulfonat-Natrium (NOBS) Gehalt von 98 %. Ausbeute NOBS: 96 %

### Vergleichsbeispiel B: Verwendung von übertrocknetem SPS, gemäß Beispiel 1b

98,1 g (0,5 mol) getrocknetes Phenolsulfonat-Natrium, hergestellt nach Beispiel 2 wurden in 150 g ISOPAR G vorgelegt und auf 120°C erwärmt. Anschließend wurden 114,8 g (0,65 mol) Nonansäurechlorid innerhalb von 30 min zugetropft und bei 120°C nachgerührt. Das dabei entstehende HCI-Gas wurde abgeleitet. Das Reaktionsgemisch wurde nach 2 h auf 80°C abgekühlt und über einen Filter abfiltriert. Das graue Reaktionsprodukt wurde 2 mal mit wenig ISOPAR G nachgewaschen und anschließend im Trockenschrank bei 110 - 130°C über Nacht getrocknet.

Telquel-Ausbeute: 129,5 g (Ausbeute 77 %) eines beige-braunen Pulvers mit einem Nonanoyloxybenzolsulfonat-Natrium (NOBS) Gehalt von 59 %. Ausbeute NOBS: 45 %.

### Vergleichsbeispiel C: Verwendung von übertrocknetem, gemahlenem SPS

Es wurde analog Vergleichsbeispiel B verfahren, das SPS jedoch direkt vor Reaktionsbeginn unter Stickstoff mittels eines Mörsers zum Feinstpulver vermahlen. Telquel-Ausbeute: 132,8 g (Ausbeute 79 %) eines beige-braunen Pulvers mit einem Nonanoyloxybenzolsulfonat-Natrium (NOBS) Gehalt von 58 %. Ausbeute NOBS: 46 %

### Beispiel 2: Zusatz von NOP

98,1 g (0,5 mol) getrocknetes Phenolsulfonat-Natrium, hergestellt nach Beispiel 1b wurden in 150 g ISOPAR G vorgelegt und auf 120°C erwärmt. Es wurden 0,7 g (3,7 mmol) N-Octylpyrrolidon hinzugefügt. Anschließend wurden 114,8 g (0,65 mol) Nonansäurechlorid innerhalb von 30 min zugetropft und bei 120°C nachgerührt. Das dabei entstehende HCI-Gas wurde abgeleitet. Das Reaktionsgemisch wurde nach 2 h auf 80°C abgekühlt und über einen Filter abfiltriert. Das weiße Reaktionsprodukt wurde 2 mal mit wenig ISOPAR G nachgewaschen und anschließend im Trockenschrank bei 110 - 130°C über Nacht getrocknet.
Telquel-Ausbeute: 165,6 g (Ausbeute 98,4 %) eines weißen Pulvers mit einem Nonanoyloxybenzolsulfonat-Natrium (NOBS) Gehalt von 97 %. Ausbeute NOBS: 95 %. Die Mutterlauge konnte ohne weitere Aufreinigung für den folgenden Ansatz genutzt werden.

### Beispiel 3: Zusatz von NMP

98,1 g (0,5 mol) getrocknetes Phenolsulfonat-Natrium, hergestellt nach Beispiel 1b wurden in 150 g ISOPAR G vorgelegt und auf 130°C erwärmt. Es wurden 0,4 g (4 mmol) N-Methylpyrrolidon hinzugefügt. Anschließend wurden 114,8 g (0,65 mol) Nonansäurechlorid innerhalb von 30 min zugetropft und bei 130°C nachgerührt. Das dabei entstehende HCI-Gas wurde abgeleitet. Das Reaktionsgemisch wurde nach 2 h auf 80°C abgekühlt und über einen Filter abfiltriert. Das weiße Reaktionsprodukt wurde 2 mal mit wenig ISOPAR G nachgewaschen und anschließend im Trockenschrank bei 110 - 130°C über Nacht getrocknet.
Telquel-Ausbeute: 164 g (Ausbeute 97,5 %) eines weißen Pulvers mit einem Nonanoyloxybenzolsulfonat-Natrium (NOBS) Gehalt von 97 %. Ausbeute NOBS: 95 %.

### Beispiel 4: Zusatz von DMF

98,1 g (0,5 mol) getrocknetes Phenolsulfonat-Natrium, hergestellt nach Beispiel 1b wurden in 150 g ISOPAR G vorgelegt und auf 120°C erwärmt. Es wurden 0,2 g (2,7 mmol) Dimethylformamid hinzugefügt. Anschließend wurden 114,8 g (0,65 mol) Nonansäurechlorid innerhalb von 30 min zugetropft und bei 120°C nachgerührt. Das dabei entstehende HCl-Gas wurde abgeleitet. Das Reaktionsgemisch wurde nach 2 h auf 80°C abgekühlt und über einen Filter abfiltriert. Das weiße Reaktionsprodukt wurde 2 mal mit wenig ISOPAR G nachgewaschen und anschließend im Trockenschrank bei 110 - 130°C über Nacht getrocknet.
Ausbeute: 165,7 g (Ausbeute 99 %) eines weißen Pulvers mit einem Nonanoyloxybenzolsulfonat-Natrium (NOBS) Gehalt von 98 %. Ausbeute NOBS: 97 %.

### Beispiel 5: Zusatz von Triethylamin

98,1 g (0,5 mol) getrocknetes Phenolsulfonat-Natrium, hergestellt nach Beispiel 1b wurden in 150 g ISOPAR G vorgelegt und auf 110°C erwärmt. Es wurden 0,2 g (2 mmol) Triethylamin hinzugefügt. Anschließend wurden 114,8 g (0,65 mol) Nonansäurechlorid innerhalb von 30 min zugetropft und bei 130°C nachgerührt. Das dabei entstehende HCI-Gas wurde abgeleitet. Das Reaktionsgemisch wurde nach 2 h auf 80°C abgekühlt und über einen Filter abfiltriert. Das weiße Reaktionsprodukt wurde zwei mal mit wenig ISOPAR G nachgewaschen und anschließend im Trockenschrank bei 110 - 130°C über Nacht getrocknet.
Telquel-Ausbeute: 161,14 g (Ausbeute 96,2 %) eines weißen Pulvers mit einem Nonanoyloxybenzolsulfonat-Natrium (NOBS) Gehalt von 97 %. Ausbeute NOBS: 93 %.

### Beispiel 6: Synthese von Lauroyloxybenzolsulfonat-Natrium

98,1 g (0,5 mol) getrocknetes Phenolsulfonat-Natrium, hergestellt nach Beispiel 1b wurden in 150 g ISOPAR G vorgelegt und auf 120°C erwärmt. Es wurden 0,7 g (3,7 mmol) N-Octylpyrrolidon hinzugefügt. Anschließend wurden 142 g (0,65 mol) Laurinsäurechlorid innerhalb von 30 min zugetropft und bei 130°C nachgerührt. Das dabei entstehende HCI-Gas wurde abgeleitet. Das Reaktionsgemisch wurde nach 2 h auf 80°C abgekühlt und über einen Filter abfiltriert. Das weiße Reaktionsprodukt wurde 2 mal mit wenig ISOPAR G nachgewaschen und anschließend im Trockenschrank bei 110 - 130°C über Nacht getrocknet.
Telquel-Ausbeute: 185,4 g (Ausbeute 98 %) eines weißen Pulvers mit einem Lauroyloxybenzolsulfonat-Natrium (LOBS) Gehalt von 98 %. Ausbeute LOBS: 96 %.

### Beispiel 7: Herstellung von 2-Methyloctanoyloxybenzolsulfonat-Natrium

98,1 g (0,5 mol) getrocknetes Phenolsulfonat-Natrium, hergestellt nach Beispiel 1b wurden in 150 g ISOPAR G vorgelegt und auf 120°C erwärmt. Es wurden 0,7 g (3,7 mmol) N-Octylpyrrolidon hinzugefügt. Anschließend wurden 114,7 g (0,65 mol) 2-Methyloctansäurechlorid innerhalb von 30 min zugetropft und bei 120°C nachgerührt. Das dabei entstehende HCI-Gas wurde abgeleitet. Das Reaktionsgemisch wurde nach 2 h auf 80°C abgekühlt und über einen Filter abfiltriert. Das weiße Reaktionsprodukt wurde 2 mal mit wenig ISOPAR G nachgewaschen und anschließend im Trockenschrank bei 110 - 130°C über Nacht getrocknet.

Telquel-Ausbeute: 160,6 g (Ausbeute 95,6 %) eines weißen Pulvers mit einem 2-Methyloctanoyloxybenzolsulfonat-Natrium Gehalt von 95 %.

## Patentansprüche

1. Verfahren zur Herstellung von Acyloxybenzolsulfonaten durch Umsetzung von Phenolsulfonaten, die einen Wassergehalt von weniger als 0,5 Gew.-% Wasser aufweisen, und Alkancarbonsäurehalogeniden, in einem aliphatischen oder aromatischen Kohlenwasserstoff, **dadurch gekennzeichnet, dass** man die Reaktion in Gegenwart von 0,01 bis 10 Gew.-% eines Katalysators in Form eines offenkettigen oder cyclischen tertiären Amins oder Carbonsäureamids durchführt.

2. Verfahren nach Anspruch 1, **dadurch gekennzeichnet, dass** man als Phenolsulfonat eine Verbindung der Formel 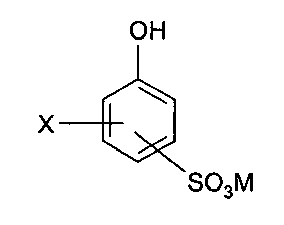 einsetzt, wobei X Wasserstoff, Halogen oder C₁-C₄-Alkyl und M ein Alkali- oder Erdalkalimetall-ion bedeutet.

3. Verfahren nach Anspruch 1, **dadurch gekennzeichnet, dass** man als Phenolsulfonat Natriumphenolsulfonat einsetzt.

4. Verfahren nach Anspruch 1, **dadurch gekennzeichnet, dass** man als Alkancarbonsäurederivat ein C₆-C₂₂-Carbonsäurechlorid einsetzt.

5. Verfahren nach Anspruch 1, **dadurch gekennzeichnet, dass** man als Alkancarbonsäurederivat und Phenolsulfonat im Molverhältnis 0,9:1 bis 2:1 umsetzt

6. Verfahren nach Anspruch 1, **dadurch gekennzeichnet, dass** man Phenolsulfonate einsetzt, die einen Wassergehalt von weniger als 0,2 Gew.-% Wasser aufweisen.

## Claims

1. A process for the preparation of acyloxybenzenesulfonates by reaction of phenolsulfonates which have a water content of less than 0.5% by weight of water, and alkanecarboxylic acid halides in an aliphatic or aromatic hydrocarbon, which comprises carrying out the reaction in the presence of from 0.01 to 10% by weight of a catalyst in the form of an open-chain or cyclic tertiary amine or carboxamide.

2. The process as claimed in claim 1, wherein the phenolsulfonate used is a compound of the formula 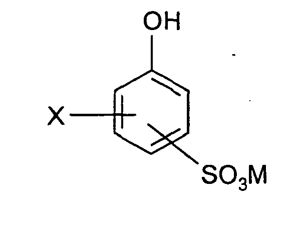 where X is hydrogen, halogen or C₁-C₄-alkyl and M is an alkali metal ion or alkaline earth metal ion.

3. The process as claimed in claim 1, wherein the phenolsulfonate used is sodium phenolsulfonate.

4. The process as claimed in claim 1, wherein the alkanecarboxylic acid derivative used is a C₆-C₂₂-carbonyl chloride.

5. The process as claimed in claim 1, wherein the alkanecarboxylic acid derivative and phenolsulfonate are reacted in the molar ratio 0.9:1 to 2:1.

6. The process as claimed in claim 1, wherein phenolsulfonates which have a water content of less than 0.2% by weight of water are used.

## Revendications

1. Procédé de préparation d'acyloxybenzène-sulfonates au moyen de la réaction de phénolsulfonates, présentant une teneur en eau inférieure à 0,5% en poids, et d'génures d'acides alcanecarboxyliques, dans un hydrocarbure aliphatique ou aromatique, **caractérisé en ce que** l'on réalise la réaction en présence de 0,01% à 10% en poids d'un catalyseur sous la forme d'une amine ou d'un amide d'acide carboxylique tertiaire à chaîne ouverte ou cyclique.

2. Procédé selon la revendication 1, **caractérisé en ce que** l'on utilise, en tant que phénolsulfonate, un composé de formule 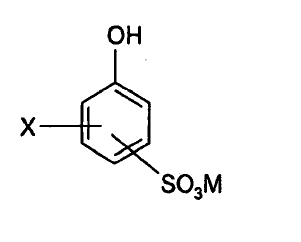 dans laquelle X représente un atome d'hydrogène, d'halogène ou un groupe alkyle en C₁ à C₄ et M représente un ion de métal alcalin ou alcalino-terreux.

3. Procédé selon la revendication 1, **caractérisé en ce que** l'on utilise, en tant que phénolsulfonate, le phénolsulfonate de sodium.

4. Procédé selon la revendication 1, **caractérisé en ce que** l'on utilise, en tant que dérivé d'acide alcanecarboxylique, un chlorure d'acide carboxylique en C₆ à C₂₂.

5. Procédé selon la revendication 1, **caractérisé en ce que** l'on fait réagir un dérivé d'acide alcanecarboxylique et un phénolsulfonate dans un rapport molaire de 0,9:1 à 2:1.

6. Procédé selon la revendication 1, **caractérisé en ce que** l'on utilise des phénolsulfonates présentant une teneur en eau inférieure à 0,2% en poids d'eau.
